# EUROPÄISCHE PATENTSCHRIFT

(11) **EP 2 711 091 B1**
(45) Veröffentlichungstag und Bekanntmachung des Hinweises auf die Patenterteilung: **02.05.2018**
(21) Anmeldenummer: 13181168.9
(22) Anmeldetag: 21.08.2013
(51) Int. Cl.: B05C 17/015, A61B 17/88

(54) **Austragsvorrichtung für fließfähige Massen**
Application device for masses capable of flowing
Dispositif d'extraction pour masses liquides

(30) Priorität: 20.09.2012 DE 102012018597
(43) Veröffentlichungstag der Anmeldung: 26.03.2014
(73) Patentinhaber: Heraeus Medical GmbH, 61273 Wehrheim (DE)
(72) Erfinder: Vogt, Sebastian, 99092 Erfurt (DE); Greiner, Clemens, 30826 Garbsen (DE); Hein, Rudolf, 31535 Neustadt (DE)
(74) Vertreter: Schultheiss & Sterzel Patentanwälte PartG mbB

(56) Entgegenhaltungen:
- EP-A1- 0 014 078
- EP-A1- 1 118 313
- EP-A1- 2 384 871
- WO-A1-95/01809
- FR-A- 1 563 664
- US-A- 3 308 990
- US-A- 4 441 629

## Beschreibung

Gegenstand der Erfindung ist eine Austragsvorrichtung zum Auspressen von fließfähigen Massen aus Kartuschen. Die Austragsvorrichtung ist insbesondere für das Austragen von Polymethylmethacrylat-Knochenzementen bestimmt.

In der Orthopädie und Unfallchirurgie werden PMMA-Knochenzemente sehr häufig zur mechanischen Fixierung von Gelenkendoprothesen verwendet. Das Auspressen von Kartuschen mit Hilfe von komprimierten Gasen ist an sich bekannt.

Bereits im Jahr 1954 wurde in der US 2,818,999 A eine Dichtungspistole vorgeschlagen, die im Griffstück eine Gaspatrone enthielt. Das komprimierte Gas der Gaspatrone drückte nach Öffnung der Patrone einen Förderkolben innerhalb einer Kartusche in Richtung Kartuschenkopf. Die Steuerung des Flusses der pastösen Masse erfolgte durch einen zentralen durch die Kartusche gehenden Stab, der die Austrittsöffnung der Kartusche verschließen konnte.

In der US 3,938,709 A von 1976 wird eine Austragsvorrichtung beschrieben, bei der durch Gasdruck eine Tube, die sich innerhalb des hohlen Pistolenkörpers befindet, ausgedrückt wird. Der Gasfluss wird dabei durch ein einfaches Stiftventil mit Feder gesteuert, das durch einen Handhebel betätigt werden kann. Eine Vorrichtung zum Ablassen des Gases war dabei nicht vorhanden. Das bedeutet, dass trotz der Unterbrechung der Gaszufuhr die Pistole das Material durch den bestehenden Restdruck nachdrückt.

Die EP 0 169 533 A2 von 1985 offenbart ein Einspritzgerät für viskose Mittel. Bei diesem fährt nach Unterbrechung der Druckgaszufuhr das Auspressen nicht fort, weil an der Abgabeöffnung ein Einspritzsteuerventil angeordnet ist, das den Strom des viskosen Mittels unterbricht. Interessant ist dabei, dass durch das Ventilelement des Abzugsgriffs einmal die Gaszufuhr und gleichzeitig der Austritt des viskosen Mittels gesteuert werden kann. Das Einspritzsteuerventil schließt, wenn keine Beaufschlagung mit komprimiertem Gas erfolgt.

In der US 4,925,061 A wird ein ähnliches System beschrieben. Bei dem jedoch das Einspritzsteuerventil über eine Stange betätigt wird, die mit dem Abzugsgriff verbunden ist.

Die EP 1 118 313 A1 offenbart eine Pistole zum Auspressen von Knochenzement. Der Antrieb erfolgt hierbei ebenfalls durch eine Gaspatrone. Wesentlich ist, dass dieses sehr komplexe System eine Stange aufweist, die dazu bestimmt ist, die im Austragsrohr enthaltene Zementrestmenge auszutreiben. Diese elegante technische Lösung ist für konventionelle Polymethylmethacrylat-Knochenzemente sehr gut geeignet. Für Kartuschensysteme zur Mischung mehrerer Komponenten mit statischem Mischer ist diese Pistole jedoch nicht verwendbar. Zudem ist die Fertigung dieser Pistole sehr aufwendig.

Die US 3 308 990 A offenbart eine Auspressvorrichtung mit einem porösen Filter, durch den ein komprimiertes CO₂-Gas als Antriebsmedium geleitet wird.

In der US 2004/0074927 A1 wird eine Auspresspistole beschrieben, welche im Wesentlichen die gleichen Merkmale wie bei US 4,925,061 A offenbart und in den Druckschriften US 2005/0230433 A1, US 2005/0247740 A1 und US 6,935,541 B1 wird die im Grunde gleiche technische Lösung vorgeschlagen, die aus der EP 0 169 533 A2 bereits bekannt ist.

Die WO 2008/109439 A1 offenbart eine durch komprimiertes Gas betriebene Austragsvorrichtung, die ein Hydraulik-Medium verwendet, auf welche das komprimierte Gas drückt.

Es ist festzustellen, dass die bis dahin bekannten Austragsvorrichtungen, die mit Gaspatronen angetrieben werden und einen komplexen mechanischen Aufbau besitzen, für die Fertigung als Einmalartikel nur bedingt oder überhaupt nicht geeignet sind. Die vorgeschlagenen technischen Lösungen lassen sich zudem nur schwer als Kunststoffspritzteile ausführen.

Bei modernen Vakuummischsystemen wird der Zement in einer Kartusche durch Vermischung des Polymerpulvers mit der Monomerflüssigkeit hergestellt. Der gebildete Zementteig wird anschließend mit Hilfe einer manuell zu betätigenden Auspressvorrichtung aus der Kartusche ausgepresst und appliziert. Der manuelle Auspressvorgang wird von vielen Anwendern wegen der dazu erforderlichen Handkraft als unangenehme empfunden. Deshalb wurden in der gattungsgemäßen DE 10 2010 019 222 A1 sowie der gattungsgemäßen DE 10 2010 019 224 B3 druckgasbetriebene Auspressvorrichtungen vorgeschlagen. Diese Vorrichtungen sind vorzugsweise aus preisgünstigem Kunststoff gefertigt und nur zum einmaligen Gebrauch bestimmt. Bei diesen Vorrichtungen wurde allgemein ein Drehventil vorgeschlagen. Diese Vorrichtungen nutzen Druckgas als Antriebsmittel, das aus Druckgaspatronen entnommen wird, die sich innerhalb der Vorrichtungen befinden. Mit Druckgas können Förderkolben von Zementkartuschen direkt beaufschlagt und damit zum Auspressen bewegt werden. Besonders vorteilhaft ist dabei die Verwendung von Druckgaspatronen mit Kohlendioxid, das in den Gaspatronen als Flüssiggas vorliegt. Bei der Verwendung solcher Austragsvorrichtungen zeigt sich, dass ein Austragen von pastösen Massen mit Hilfe des verdampfenden Kohlendioxids möglich ist. Der durch die von verdampfenden Kohlendioxid erzeugte Druck bei Raumtemperatur von ungefähr 55 bar reicht völlig aus, um hochviskose Knochenzemente, wie Palacos® R+G, aus Kartuschen auszupressen.

Nachteilig ist jedoch, dass mitunter nur ein Teil des flüssigen Kohlendioxids in der Austragsvorrichtung verdampft und flüssiges Kohlendioxid bis zu dem Förderkolben vordringt. Dadurch gibt es infolge der unkontrollierten Nachverdampfung in dem Raum zwischen dem Förderkolben und dem Ventilelement Druckspitzen, die zu unerwünschten kurzfristigen Beschleunigungen der Förderkolben führen. Dadurch wird die präzise Regulierung des Austragsvorgangs gestört.

Die Aufgabe der Erfindung besteht also darin, die Nachteile des Stands der Technik zu überwinden. Der Erfindung liegt dabei die Aufgabe zu Grunde, eine möglichst einfache, unkompliziert zu handhabende Austragsvorrichtung für fließfähige Massen zu entwickeln. Diese soll unter Verwendung von flüssigem Kohlendioxid oder einem anderen leicht verdampfbaren Flüssiggas eine präzise Regulierung des Austragsvorgangs von fließfähigen Massen ermöglichen. Unter dem Begriff fließfähige Massen werden sowohl flüssige, zähflüssige als auch hochviskose, zähe Massen, die nur bei Druckbeaufschlagung fließen, verstanden. Das Austragen der fließfähigen Massen soll durch komprimiertes verflüssigtes Gas, insbesondere durch Kohlendioxid erfolgen, das einer Gaskartusche oder einer Gaspatrone entnommen wird, die sich in der Vorrichtung befindet oder an diese anschließbar ist. Bei der Verwendung von Kohlendioxid-Patronen liegt in der Gaskartusche das Kohlendioxid in flüssiger Form vor. Die Vorrichtung soll aus möglichst wenigen Teilen bestehen, die durch kostengünstiges Spritzgießen mit konventionellen Kunststoffen hergestellt werden können. Angestrebt wird weiterhin, dass die Austragsvorrichtung als einmal zu gebrauchender Wegwerfartikel geeignet ist.

Daher wurde die Aufgabe der Erfindung gelöst durch eine Austragsvorrichtung zum Austragen fließfähiger Massen aus Kartuschen, wobei die Austragsvorrichtung einen Grundkörper, ein Anschlusselement für eine Gaspatrone am Grundkörper, ein Ventilelement zum Regulieren eines Gasstroms aus der Gaspatrone und einen gasdurchlässigen Kanal aufweist, der das Anschlusselement mit dem Ventilelement verbindet, wobei in dem gasdurchlässigen Kanal zumindest ein Verdampfungsbehälter angeordnet ist, so dass der Gasstrom aus der Gaspatrone zumindest bereichsweise durch den Verdampfungsbehälter geleitet wird, wobei der gasdurchlässige Kanal zwei Verbindungselemente um-fasst, die mit dem Verdampfungsbehälter verbunden sind, so dass der Gasstrom aus der Gaspatrone zwischen den Verbindungselementen durch den Verdampfungsbehälter strömt, und wobei die Verbindungselemente nicht an gegenüberliegenden Wandungen des Verdampfungsbehälters mit dem Verdampfungsbehälter verbunden sind.

Dabei kann vorgesehen sein, dass der Verdampfungsbehälter einen größeren Querschnitt als der gasdurchlässige Kanal aufweist, bevorzugt einen zumindest doppelt so großen Querschnitt, besonders bevorzugt einen zumindest zehnmal so großen Querschnitt.

Ferner kann vorgesehen sein, dass der Verdampfungsbehälter ein Volumen aufweist, das wenigstens halb so groß, bevorzugt wenigstens gleich groß wie das Volumen eines flüssigen Druckgases in der Gaspatrone ist, die zum Anschließen an das Anschlusselement vorgesehen ist oder die an das Anschlusselement angeschlossen ist.

Durch diese Aufbauvarianten kann sichergestellt werden, dass flüssige Bestandteile des Gasstroms im Verdampfungsbehälter ausreichend Raum haben, um sich von dem Gasstrom zu trennen und in den gasförmigen Zustand überzugehen.

Die Erfindung sieht vor, dass der gasdurchlässige Kanal zwei Verbindungselemente umfasst, die mit dem Verdampfungsbehälter verbunden sind, so dass der Gasstrom aus der Gaspatrone zwischen den Verbindungselementen durch den Verdampfungsbehälter strömt.

Hierdurch kann der Verdampfungsbehälter ausgetauscht oder nachträglich eingepasst werden und dadurch an das System, insbesondere an das Volumen der Gaspatrone angepasst werden. Aber auch wenn die Verbindungselemente fest und unlösbar mit dem Verdampfungsbehälter verbunden sind ergibt sich der Vorteil, dass eine stabile Verbindung und eine unaufwendige Fertigung der erfindungsgemäßen Austragsvorrichtung möglich sind.

Dabei ist vorgesehen, dass die Verbindungselemente nicht an gegenüberliegenden Wandungen des Verdampfungsbehälters mit dem Verdampfungsbehälter verbunden sind, wobei bevorzugt die Verbindungselemente auf der gleichen Seite mit dem Verdampfungsbehälter verbunden sind.

Durch diese Maßnahme wird sichergestellt, dass sich die flüssigen Bestandteile des Gasstroms nicht aufgrund ihrer Trägheit einfach gerade durch den gasdurchlässigen Kanal hindurchbewegen und so bis zum Ventilelement oder weiter gelangen. Je stärker die Umleitung des Gasstroms in dem Verdampfungsbehälter ist und je besser sich die Erdanziehung zur Trennung ausnutzen lässt, desto effektiver kann eine Trennung des Gasstroms von den flüssigen Bestandteilen erfolgen. Aus dem gleichen Grund kann erfindungsgemäß vorgesehen sein, dass der gasdurchlässige Kanal gewunden ist, insbesondere zumindest zwei Richtungswechsel aufweist.

Ferner kann vorgesehen sein, dass zwischen den Verbindungselementen eine gasundurchlässige Wandung im Grundkörper angeordnet ist.

Auch die Wandung soll dazu führen, dass eine Trennung des Gasstroms von den flüssigen Bestandteilen möglichst effizient erfolgt. Zudem kann dadurch das flüssige Druckgas nicht direkt vom ersten Verbindungselement zum zweiten Verbindungselement gelangen. Das flüssige Druckgas wird zwangsweise in den Verdampfungsbehälter geleitet.

Bei einer besonders bevorzugten Ausführung der Erfindung kann vorgesehen sein, dass bei einer normalen und so vorgesehenen Verwendung der Austragsvorrichtung zum Austragen einer fließfähigen Masse aus einer Kartusche der Verdampfungsbehälter derart in der Austragsvorrichtung angeordnet ist, dass die beiden Öffnungen des gasdurchlässigen Kanals in den Verdampfungsbehälter nicht bodenseitig mit dem Verdampfungsbehälter verbunden sind, so dass flüssige Bestandteile des Gasstroms sich bodenseitig im Verdampfungsbehälter sammeln, wobei bevorzugt die Öffnungen des gasdurchlässigen Kanals in den Verdampfungsbehälter im oberen Bereich des Verdampfungsbehälters angeordnet sind, besonders bevorzugt in der Deckwandung des Verdampfungsbehälters angeordnet sind.

Durch diese Anordnung kann die Gravitation zur Trennung der flüssigen Bestandteile von dem Gasstrom genutzt werden. Zudem können die flüssigen Bestandteile so nicht in Richtung des Drehventils durch den gasdurchlässigen Kanal fließen.

Des Weiteren kann vorgesehen sein, dass die Austragsvorrichtung ein Griffstück aufweist, wobei der Verdampfungsbehälter zumindest bereichsweise in dem Griffstück angeordnet ist, bevorzugt der Verdampfungsbehälter im Wesentlichen im Griffstück angeordnet ist, besonders bevorzugt der Verdampfungsbehälter im Griffstück angeordnet ist.

Die Unterbringung des Verdampfungsbehälters oder eines Teils davon stellt sicher, dass die Austragsvorrichtung immer so gehalten wird, dass die Gravitation ihre Wirkung zur Trennung der flüssigen Bestandteile vom Gasstrom tun kann. Zudem erleichtert das Griffstück die Bedienbarkeit der Austragsvorrichtung.

Zur Bereitstellung einer möglichst kompletten Austragvorrichtung kann vorgesehen sein, dass die Austragsvorrichtung eine Gaspatrone beinhaltet, vorzugsweise eine Druckgaspatrone, insbesondere eine mit flüssigem Kohlendioxid gefüllte Gaspatrone.

Gemäß einer Weiterbildung der Erfindung kann vorgesehen sein, dass am Anschlusselement ein Dichtungselement zum Anschließen der Gaspatrone vorgesehen ist.

Durch das Dichtungselement kann ein ungewollter Verlust des strömenden Gases an der Verbindung vermieden werden.

Es kann ferner vorgesehen sein, dass am Grundkörper eine Adaptereinheit zum Anschließen von Kartuschen angeordnet ist, wobei in dem Grundkörper ein zweiter und ein dritter gasdurchlässiger Kanal vorgesehen ist, die das Ventilelement mit der Außenseite der Adaptereinheit verbinden.

Mit diesem Aufbau können unterschiedliche Kartuschen mit der Austragsvorrichtung verbunden werden. Zudem erhöhen die zusätzlichen gasdurchlässigen Kanäle den Bedienkomfort der Austragsvorrichtung.

Dabei kann vorgesehen sein, dass zumindest ein Überdruckventil und/oder zumindest ein Sterilfilter mit dem dritten Kanal verbunden ist oder über das Ventilelement verbindbar ist und das Überdruckventil und/oder der Sterilfilter über eine Öffnung im Grundkörper mit der umgebenden Atmosphäre verbunden ist, wobei die Öffnung nicht in der Adaptereinheit angeordnet ist.

Überdruckventile und Sterilfilter tragen zur Sicherheit bei der Bedienung und der Anwendung erfindungsgemäßer Austragsvorrichtungen bei.

Bevorzugte Austragsvorrichtungen können vorsehen, dass das Ventilelement einen ersten Gaskanal zum Regulieren des Druckgasstroms aufweist und bevorzugt zusätzlich einen zweiten Gaskanal aufweist, der so angeordnet ist, dass bei Betätigung des Ventilelements eine Unterbrechung des ersten Gaskanals erfolgt und der zweite Gaskanal geöffnet ist, so dass der zweite Gaskanal über den Grundkörper gasdurchlässig mit der umgebenden Atmosphäre verbunden ist.

Durch diese Maßnahmen wird der Bedienkomfort der Austragsvorrichtung gesteigert.

Auch kann vorgesehen sein, dass der der Verdampfungsbehälter und/oder der Grundkörper aus Kunststoff besteht, bevorzugt aus Polyethylen.

Ferner kann vorgesehen sein, dass die Austragsvorrichtung zu mehr als 90% aus Kunststoff besteht, vorzugsweise aus Polyethylen, Polyoxymethylen, Polyamid oder Teflon besteht. Der Verdampfungsbehälter lässt sich wirtschaftlich sinnvoll durch Blasformen herstellen. Daneben kann die Herstellung auch durch Spritzgießen erfolgen.

Hierdurch kann eine besonders kostengünstige Fertigung der Austragsvorrichtung erreicht werden.

Im Rahmen der Erfindung sind auch alle sonstigen denkbaren Werkstoffe anwendbar, die zur Herstellung von druckfesten Verdampfungsbehältern geeignet sind. So lassen sich zum Beispiel Verdampfungsbehälter auch aus Metall und dabei bevorzugt aus Aluminium oder Aluminiumlegierungen herstellen.

Eine vorteilhafte Ausgestaltung erfindungsgemäßer Austragsvorrichtungen besteht darin, dass gegebenenfalls mindestens ein Überdruckventil (Sicherheits-Überdruckventil) mit wenigstens einem gasdurchlässigen Kanal verbunden ist, der die Außenseite des Kartuschenanschlussstücks mit dem Ventilelement gasdurchlässig verbindet, wobei das Überdruckventil mit der die Außenseite des Grundkörpers umgebenden Atmosphäre gasdurchlässig verbunden ist. Das Überdruckventil kann zum Beispiel als einfaches Kugel-Überdruckventil ausgebildet sein. Es öffnet erst ab einen zuvor festgelegten Innendruck. Das Überdruckventil dient dazu, im Falle einer zu hohen Druckbeanspruchung die Berstsicherheit der Austragsvorrichtung durch Abblasen des überschüssigen Gases in die umgebende Atmosphäre zu gewährleisten.

Als besonders vorteilhaft hat es sich dabei erwiesen, wenn zwei Überdruckventile nacheinander mit dem gasdurchlässigen Kanal oder besonders bevorzugt dem zweiten gasdurchlässigen Kanal verbunden sind, so dass auch im Falle des sehr unwahrscheinlichen Versagens von einem Überdruckventil die Berstsicherheit der Vorrichtung durch die Funktion des zweiten Überdruckventils gewährleistet ist.

Eine weitere vorteilhafte Ausgestaltung der Erfindung besteht darin, dass das Ventilelement einen ersten Gaskanal zum Regulieren des Druckgasstroms hat und einen zweiten Gaskanal, der so angeordnet ist, dass bei Betätigung des Ventilelements mit der Unterbrechung des ersten Gaskanals der zweite Gaskanal geöffnet ist, der mit einem im Ventilelement angeordneten dritten Gaskanal verbunden ist, der gasdurchlässig mit der umgebenden Atmosphäre verbunden ist. Das bedeutet, dass nach Schließen des ersten Gaskanals durch den zweiten Gaskanal und dritten Gaskanal das hinter dem Ventilelement unter Druck stehende Gas in die umgebende Atmosphäre abgeblasen wird. Dadurch wird die Druckbeaufschlagung des Förderkolbens der Kartusche sofort beendet und ein Nachlaufen des Zementteigs wird verhindert. Weiterhin erhöht sich dadurch die Handhabungssicherheit der Austragsvorrichtung beträchtlich.

Die Aufgaben der Erfindung werden auch gelöst durch die Verwendung einer erfindungsgemäßen Austragsvorrichtung zum Auspressen einer fließfähigen Masse aus einer Kartusche, bei dem ein verdampfendes flüssiges Gas aus einer Gaspatrone in den Verdampfungsbehälter geleitet wird und das verdampfte Gas vom Verdampfungsbehälter über ein regulierbares Ventilelement zu einem Förderkolben der Kartusche geleitet wird, wobei der Gasdruck den Förderkolben vortreibt und die fließfähige Masse durch den Vortrieb des Förderkolbens aus der Kartusche ausgetrieben wird.

Der Erfindung liegt die überraschende Erkenntnis zugrunde, dass durch einen Verdampfungsbehälter, der zwischen der Gaspatrone und dem Ventilelement im Weg des Gases angeordnet ist, der Gasstrom durch Betätigung eines Ventilelements problemlos und kontinuierlich reguliert werden kann, weil flüssiges Druckgas, das aus der Gaspatrone ausdringt, vor dem Ventilelement im Verdampfungsbehälter verdampft wird. Dadurch wird der Durchtritt von flüssigem Druckgas durch das Ventilelement vermieden. Es kann somit kein flüssiges Druckgas im Raum zwischen dem Förderkolben und dem Ventilelement nachverdampfen, wodurch unerwünschte Druckspitzen vermieden werden können. Ein ungewolltes Vortreiben des Förderkolbens in der Kartusche bei geschlossenem Ventilelement kann so erfindungsgemäß ebenfalls vermieden werden.

Vorteilhaft ist weiterhin, dass durch Vermeidung von Druckspitzen die Druckbelastung für die Austragsvorrichtung verringert wird und damit die Gefahr eines Zerberstens der Austragsvorrichtung beziehungsweise von Teilen davon reduziert wird. Ein weiterer Vorteil besteht darin, dass bei gleichzeitiger Verwendung von Überdruckventilen (Sicherheits-Überdruckventile) in der Austragsvorrichtung infolge des Nichtauftretens von Druckspitzen deutlich weniger Druckgas über die Überdruckventile abgeblasen wird als bei einer Austragsvorrichtung ohne Verdampfungsbehälter. Dadurch steht mehr Druckgas zum Austragen der fließfähigen Massen zur Verfügung. Es können dadurch kleinere Gaspatronen mit einer geringeren Füllmenge an flüssigem Druckgas verwendet werden.

Eine erfindungsgemäße Austragsvorrichtung kann beispielsweise aufgebaut sein aus
a) einem Kunststoffkörper,
b) einem Anschlusselement für eine Gaspatrone am Kunststoffkörper,
c) eine Gaspatrone mit Flüssiggas,
d) einem Dichtungselement für eine Gaspatrone im Anschlusselement der Gaspatrone,
e) einem Öffnungsmittel für eine Gaspatrone im Anschlusselement,
f) einem gasdurchlässigen Kanal vom Anschlusselement der Gaspatrone bis zu einem Verbindungselement 1,
g) einem gasdurchlässigen Kanal vom Verbindungselement 2 bis zu dem Ventilelement,
h) einem Ventilelement,
i) eine Adaptereinheit am Kunststoffkörper zum Konnektieren von Kartuschen,
j) wenigstens zwei gasdurchlässige Kanäle, welche die Außenseite der Adaptereinheit mit dem Ventilelement gasdurchlässig verbinden,
wobei ein Verdampfungsbehälter mit mindestens zwei Öffnungen in dem gasdurchlässigen Kanälen angeordnet ist, wobei eine erste Öffnung gasdurchlässig mit dem Verbindungselement und eine zweite Öffnung gasdurchlässig mit dem Verbindungselement verbunden sind.

Im Folgenden werden Ausführungsbeispiele der Erfindung anhand von zwei schematisch dargestellten Figuren erläutert, ohne jedoch dabei die Erfindung zu beschränken. Dabei zeigt:
- Figur 1:: eine schematische Querschnittansicht einer erfindungsgemäßen Austragsvorrichtung; und
- Figur 2:: eine schematische Querschnittansicht einer weiteren erfindungsgemäßen Austragsvorrichtung ohne Kartusche und ohne Gaspatrone.

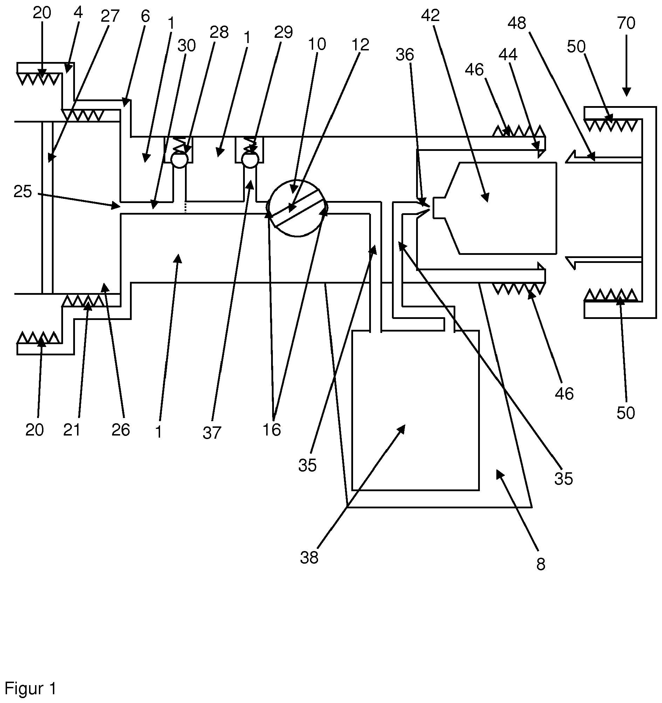

Figur 1 zeigt eine schematische Querschnittansicht einer erfindungsgemäßen Austragsvorrichtung mit einem Grundkörper 1 an dessen Vorderseite ein erstes Kartuschenanschlussstück 4 mit großem Durchmesser für Kartuschen mit großem Durchmesser und ein zweites Kartuschenanschlussstück 6 mit kleinerem Durchmesser für Kartuschen mit kleinerem Durchmesser angeordnet sind. An den Zylinderinnenwänden der Kartuschenanschlussstücke 4, 6 sind Fixierungsvorrichtungen 20, 21 in Form von Innengewinden zur Fixierung von Kartuschen mit Außengewinden (nicht gezeigt) vorgesehen. Die Fixierungsvorrichtungen 20, 21 können auch durch Gewinde, Gewindeausschnitte oder Zapfen zum Arretieren der Kartuschen in den Kartuschenanschlussstücken 4, 6 realisiert sein.

An der Unterseite beziehungsweise der Bodenseite der Austragsvorrichtung ist ein Pistolengriff 8 vorgesehen, mit dem die Austragsvorrichtung in einer Hand gehalten werden kann. Die Unterseite beziehungsweise die Bodenseite der Austragsvorrichtung ist dabei die Seite der Austragsvorrichtung, die bei einer normalen und so vorgesehenen Verwendung der Austragsvorrichtung zum Austragen einer fließfähigen Masse aus einer Kartusche in Richtung Boden also nach unten weist. Die Anordnung des Pistolengriffs 8 stellt die bodenseitige Richtung dabei klar.

In der rückseitigen Wand des zweiten, inneren Kartuschenanschlussstücks 6 befinden sich zwei Öffnungen 25, die in zwei frontseitigen gasdurchlässigen Kanälen 30 im Grundkörper 1 münden. Die frontseitigen gasdurchlässigen Kanäle 30 sind in der Bildebene nach Figur 1 hintereinander angeordnet. Der vordere gasdurchlässige Kanal 30 erstreckt sich bis zu einem Ventilelement 10, das als Drehventil mit zylindrischem Ventilsitz ausgebildet ist. In Form eines T-Stücks ist der vordere Kanal mit einem Überdruckventil 29 verbunden. Der hintere gasdurchlässige Kanal 30, der sich eigentlich nicht in der Schnittebene befindet, aber zur Veranschaulichung der Gesamtheit des Aufbaus der Austragsvorrichtung dennoch eingezeichnet ist, knickt nach oben ab (in Figur 1 durch die gepunktete Linie angedeutet) und mündet dort über ein zweites Überdruckventil 28 nach außen in die Umgebung, das heißt in die, die Austragsvorrichtung umgebende Atmosphäre. In den Verbindungen, die die frontseitigen gasdurchlässigen Kanäle 30 mit der Umgebung verbinden, können Sterilfilter (nicht gezeigt) angeordnet sein, um sicherzustellen, dass keine Kontamination des Inneren der Austragsvorrichtung auftreten kann.

In dem Kartuschenanschlussstück 6 mit dem kleineren Durchmesser ist eine Kartusche 26 fixiert, die dicht mit dem Kartuschenanschlussstück 6 abschließt. Dadurch wird gewährleistet, dass ein Gasdruck, der durch eine der Öffnungen 25 auf den Boden der fixierten Kartusche 26 gerichtet wird, auch auf einen auf der Seite des Kartuschenbodens angeordneten Förderkolben 27 der Kartusche 26 wirken kann, und nicht zwischen der Kartusche 26 und dem Kartuschenanschlussstücke 6 entweicht. Hierzu können Dichtungselemente (nicht gezeigt) in den Kartuschenanschlussstücken 4, 6 und/oder an den Kartuschen 26, insbesondere an den Kartuschenböden, vorgesehen sein. Es kann aber auch ausreichend sein, eine dichte Fixierungsvorrichtung 20, 21 vorzusehen, die beispielsweise durch ein Gewinde realisiert sein kann.

In einer zylindrischen Öffnung im Grundkörper 1 befindet sich der Drehventilkörper 10 mit bereichsweise rotationssymmetrischer Geometrie, der um seine Symmetrieachse (senkrecht zur in Figur 1 dargestellten Schnittebene) drehbar im Grundkörper 1 gelagert ist. In dem Ventilelement 10 befindet sich ein erster Gaskanal 12, der sich gerade durch den Drehventilkörper 10 erstreckt. Um 20° versetzt zu den Öffnungen des Gaskanals 12 im Zylindermantel des Drehventilkörpers 10 befinden sich Dichtnocken 16, die der Abdichtung des gasdurchlässigen Kanals 30 und eines rückseitigen gasdurchlässigen Kanals 35 im Grundkörper 1 dienen, wenn der Drehventilkörper 10 in die geschlossene Position gedreht ist, die in Figur 1 gezeigt ist.

Im hinteren Teil des Grundkörpers 1 ist ein rückseitiger gasdurchlässiger Kanal 35 angeordnet, der in eine Öffnung im Ventilsitz zum Drehventilkörper 10 mündet. Mit den Dichtnocken 16 sind die Öffnungen zu den gasdurchlässigen Kanälen 30, 35 im Ventilsitz des Drehventils 10 gasdicht und bis zu einem Druck von siebzig bar Druckdicht verschließbar. Auf der, der Rückseite der Austragsvorrichtung zugewandten Seite des Grundkörpers 1 mündet der rückseitige gasdurchlässige Kanal 35 in einen hohlen, beidseitig geöffneten Dorn 36 mit einem Durchlass.

Mit einem zusätzlich vorgesehenen Ablasskanal (nicht gezeigt) kann ein im Bereich der Kartuschenanschlussstücke 4, 6 auf dem Förderkolben 27 der angeschlossenen Kartusche 26 lastende Gasdruck abgelassen werden. Dazu kann im Drehventilkörper 10 ein weiterer Gaskanal (nicht gezeigt) angeordnet sein, über den bei geeigneter Stellung des Drehventilkörpers 10 eine dritte Öffnung neben den Öffnungen 25 mit der einem zusätzlichen frontseitigen dritten Gaskanal (nicht gezeigt) verbunden werden kann. Zum Verschließen der Öffnungen im Ventilsitz hierzu wären in diesem Fall bevorzugt zusätzliche Dichtnocken (nicht gezeigt) vorgesehen. Im Drehventilkörper 10 sind dann zwei entlang der Symmetrieachse des Drehventilkörpers 10 versetzte Gaskanäle 12, die zusätzlich um diese Symmetrieachse gegeneinander verdreht sind, angeordnet. Die versetzten Gaskanäle 12, von denen in Figur 1 nur einer gezeigt ist, sind dann derart im Drehventilkörper 10 angeordnet und die gasdurchlässigen Kanäle 30, 35 derart im Grundkörper 1 angeordnet, dass bei zwei Stellungen des Drehventilkörpers 10, die durch eine Drehung des Drehventilkörpers 10 gegen den Grundkörper 1 im Ventilsitz einstellbar sind, ein frontseitiger gasdurchlässigen Kanal 30 mit dem rückseitigen gasdurchlässigen Kanal 35 über den Gaskanal 12 oder den Ablasskanal (nicht gezeigt) miteinander verbunden sind, wobei alle anderen gasdurchlässigen Kanäle 30, 35 durch die Dichtnocken 16 des Drehventilkörpers 10 druckdicht geschlossen sind. Die Breite der vorderen Öffnung 25 zum Drehventilkörper 10, die in den frontseitigen gasdurchlässigen Kanal 30 mündet, kann auch dazu ausreichen, dass jeder der Gaskanäle 12 in dem Drehventilkörper 10 mit dem dann einzigen frontseitigen gasdurchlässigen Kanal 30 verbindbar ist.

Am Drehventilkörper 10 ist auf einer Seite, die aus dem Grundkörper 1 herausragt, ein Schlüsselelement (nicht gezeigt) angeordnet, in den ein Bedienelement (nicht gezeigt) mit passendem Gegenstück zum Drehen des Drehventils 10 im Ventilsitz des Grundkörpers 1 greift.

Der Dorn 36 ragt in einen Hohlraum im hinteren Teil des Grundkörpers 1, der zur Aufnahme einer Gaspatrone 42 geeignet ist. Die Gaspatrone 42 ist mit flüssigem Kohlendioxid gefüllt. Der Dorn 36 dient dazu, eine Gaspatrone 42, deren Öffnungsseite auf den Dorn 36 gedrückt wird, zu öffnen. Am hinteren Ende des Hohlraums ist innenseitig eine Arretierungsvorrichtung 44 und außenseitig am Grundkörper 1 ein Befestigungsmittel 46 in Form eines Außengewindes angeordnet. Die Arretierungsvorrichtung 44 dient dazu, eine ungewollte Bewegung einer Verschlusskappe 70 zu verhindern, mit der die Austragsvorrichtung rückseitig verschließbar ist. Die Arretierungsvorrichtung 44 kann dazu in Arretierungshaken 48 greifen, die an der Verschlusskappe 70 angeordnet sind. Das Befestigungsmittel 46 dient dazu, die Verschlusskappe 70 mit dem Grundkörper 1 sicher zu verbinden, dabei die Gaspatrone 42 auf den Dorn 36 zu drücken und so zu öffnen. Dazu ist an der Verschlusskappe 70 ein Befestigungsmittel 50 in Form eines Innengewindes vorgesehen.

In dem rückseitigen gasdurchlässigen Kanal 35 ist ein Verdampfungsbehälter 38 angeordnet, der sich im Pistolengriff 8 der Austragsvorrichtung befindet. Der rückseitige gasdurchlässige Kanal 35 ist dazu nach unten in Richtung des Pistolengriffs 8 an zwei Stellen abgeknickt, so dass die beiden nach unten weisenden Enden des rückseitigen gasdurchlässigen Kanals 35 in einer Deckelfläche in den Verdampfungsbehälter 38 münden. Das aus der Gaspatrone 42 entweichende Kohlendioxid wird beim Passieren des rückseitigen gasdurchlässigen Kanals 35 also durch den Verdampfungsbehälter 38 geleitet. Statt Kohlendioxid kann auch ein anderes unter Druck verflüssigtes Gas verwendet werden, das zur Erzeugung des Gasstroms verdampft wird.

Der Verdampfungsbehälter 38 hat einen zehnmal größeren Querschnitt als der rückseitige gasdurchlässige Kanal 35 und ein Volumen das etwa so groß ist, wie das Volumen des flüssigen Kohlendioxids in einer neuen (noch nicht angebrochenen) Gaspatrone 42.

Wenn flüssiges Kohlendioxid aus der Gaspatrone 42 austritt gelangt es durch den rückseitigen gasdurchlässigen Kanal 35 in den Verdampfungsbehälter 38 und sammelt sich dort am Boden des Verdampfungsbehälters 38. Im Verdampfungsbehälter 38 verdampft das flüssige Kohlendioxid und wird durch den rückseitigen gasdurchlässigen Kanal 35 bis zum Ventilelement 10 weitergeleitet. Wenn das Ventilelement 10 sich in der geöffneten Stellung befindet (nicht in Figur 1 gezeigt), dann durchströmt das vollständig verdampfte Kohlendioxid den Gaskanal 12 im Ventilelement 10 und anschließend durch den verbundenen frontseitigen gasdurchlässigen Kanal 30 bis zur Bodenseite der Kartusche 26 im Kartuschenanschlussstück 6. Dort drückt der Gasdruck des Kohlendioxids auf den Förderkolben 27 und treibt diesen in der Kartusche 26 nach vorne und dadurch den Kartuscheninhalt (eine fließfähige Masse, beispielsweise eine Knochenzement) nach vorne aus der Kartusche 26 heraus, wo der Kartuscheninhalt appliziert werden kann.

Wenn das Ventilelement 10 geschlossen wird (in Figur 1 gezeigte Position) wird sich der auf dem Förderkolben 27 lastende Druck sehr schnell entspannen und so der Vortrieb des Förderkolbens 27 sehr schnell gestoppt. Durch den Verdampfungsbehälter 38 wird dabei auf einfache Weise und kostengünstige Weise sichergestellt, dass kein flüssiges Kohlendioxid durch die gasdurchlässigen Kanäle 30, 35 und durch den Gaskanal 12 bis zum Kartuschenanschlussstück 4, 6 oder bis in den frontseitigen gasdurchlässigen Kanal 30 gelangt und dieses flüssige Kohlendioxid stoßweise verdampft, sein Volumen ausdehnt und dabei einen Druckstoß erzeugt, der zu einem ungewollten ungleichmäßigen Vortrieb des Förderkolbens 27 führt und zu einem ungewollten Ausblasen des Gases durch die Überdruckventile 28, 29.

Die Kartusche 26 und die Gaspatrone 42 können auch separate Teile sein, die nicht zur Austragsvorrichtung gehören.

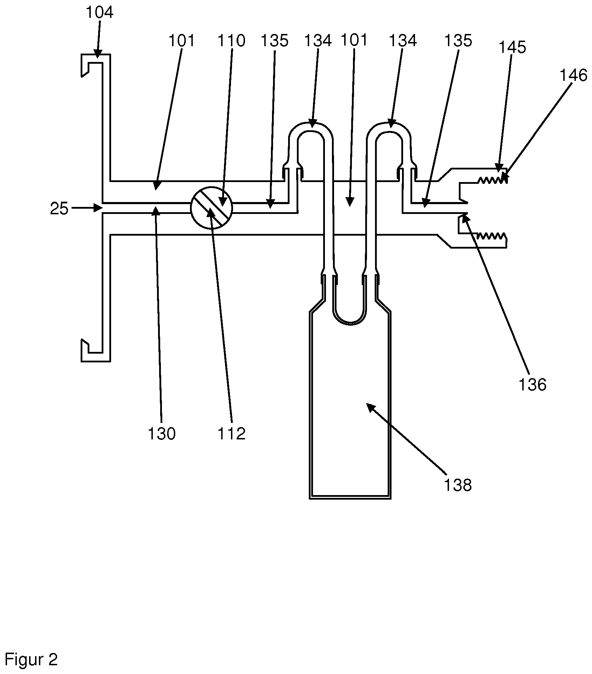

Figur 2 zeigt eine schematische Querschnittansicht eines weiteren Ausführungsbeispiels einer erfindungsgemäßen Austragsvorrichtung. An einem Grundkörper 101 aus Polyethylen befindet sich ein Kartuschenanschlussstück 104 zum Anschließen einer Kartusche mit einem bodenseitigen Förderkolben. In dem Grundkörper 101 befindet sich ein frontseitiger (zum Kartuschenanschlussstück 104 gerichteter) gasdurchlässiger Kanal 130, der sich von einem Ventilelement 110 bis zu einer Öffnung 25 im Kartuschenanschlussstück 104 erstreckt.

An dem, dem Kartuschenanschlussstück 104 gegenüberliegenden, rückseitigen Ende der Austragsvorrichtung ist ein Anschluss 145 für eine Gaspatrone angeordnet. In dem Anschluss 145 ist ein Innengewinde 146 als Befestigungsmittel vorgesehen, in das ein Außengewinde an einer passenden Gaspatrone (nicht gezeigt) geschraubt werden kann. Im Anschluss 145 ist am Grundkörper 101 ein hohler Dorn 136 als Anschlusselement vorgesehen, mit dem die Gaspatrone geöffnet wird, wenn sie in das Innengewinde 146 geschraubt wird.

Der hohle Dorn 136 ist über zwei Teile eines rückseitigen gasdurchlässigen Kanals 135, zwei Schläuche 134 und einen Verdampfungsbehälter 138 mit dem Ventilelement 110 verbunden, die einen gasdurchlässigen Kanal bilden. In dem Ventilelement 110 befindet sich ein Gaskanal 112, der in einer geeigneten Stellung des Ventilelements 110 die Verbindung beziehungsweise den Kanal, der durch den rückseitigen gasdurchlässigen Kanal 135, die Schläuche 134 und den Verdampfungsbehälter 138 gebildet wird, mit dem frontseitigen gasdurchlässigen Kanal 130 gasdurchlässig verbindet.

Der Verdampfungsbehälter 138 hat wie der Verdampfungsbehälter 38 nach Figur 1 die Aufgabe flüssige Bestandteile, die in die Verbindung beziehungsweise den Kanal gelangen, zu verdampfen und damit zu verhindern, dass diese flüssigen Flüssiggasbestandteile zum Kartuschenanschlussstück 104 oder in der frontseitigen gasdurchlässigen Kanal 130 gelangen.

Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein.

### Bezugszeichenliste

- 1, 101: Grundkörper
- 4: Kartuschenanschlussstück mit großem Durchmesser
- 6: Kartuschenanschlussstück mit kleinem Durchmesser
- 8: Pistolengriff
- 10, 110: Ventilelement / Drehventilkörper
- 12,112: Gaskanal
- 16: Dichtnocke
- 20, 21: Fixierungsvorrichtung
- 25: Öffnung im Kartuschenanschlussstück
- 26: Kartusche
- 27: Förderkolben
- 28, 29: Überdruckventil
- 30, 35: Gasdurchlässiger Kanal
- 36, 136: Hohler Dorn
- 37: Ablasskanal
- 38, 138: Verdampfungsbehälter
- 42: Gaspatrone
- 44: Arretierungsvorrichtung
- 46, 146: Befestigungsmittel
- 48: Arretierungshaken
- 50: Befestigungsmittel
- 70: Verschlusskappe
- 104: Kartuschenanschlussstück
- 134: Schlauch
- 130, 135: Gasdurchlässiger Kanal
- 145: Anschluss

## Patentansprüche

1. Austragsvorrichtung zum Austragen fließfähiger Massen aus Kartuschen (26),
wobei die Austragsvorrichtung
einen Grundkörper (1, 101),
ein Anschlusselement (36, 136) für eine Gaspatrone (42) am Grundkörper (1, 101),
ein Ventilelement (10, 110) zum Regulieren eines Gasstroms aus der Gaspatrone (42) und
einen gasdurchlässigen Kanal (35, 134, 135) aufweist, der das Anschlusselement (36, 136, 145) mit dem Ventilelement (10, 110) verbindet, **dadurch gekennzeichnet, dass**
in dem gasdurchlässigen Kanal (35, 134, 135) zumindest ein Verdampfungsbehälter (38, 138) angeordnet ist, so dass der Gasstrom aus der Gaspatrone (42) zumindest bereichsweise durch den Verdampfungsbehälter (38, 138) geleitet wird, wobei der gasdurchlässige Kanal (35, 134, 135) zwei Verbindungselemente umfasst, die mit dem Verdampfungsbehälter (38, 138) verbunden sind, so dass der Gasstrom aus der Gaspatrone (42) zwischen den Verbindungselementen durch den Verdampfungsbehälter (38, 138) strömt, und wobei die Verbindungselemente nicht an gegenüberliegenden Wandungen des Verdampfungsbehälters (38, 138) mit dem Verdampfungsbehälter (38, 138) verbunden sind.

2. Austragsvorrichtung nach Anspruch 1, **dadurch gekennzeichnet, dass** der Verdampfungsbehälter (38, 138) einen größeren Querschnitt als der gasdurchlässige Kanal (35, 134, 135) aufweist, bevorzugt einen zumindest doppelt so großen Querschnitt, besonders bevorzugt einen zumindest zehnmal so großen Querschnitt.

3. Austragsvorrichtung nach Anspruch 1 oder 2, **dadurch gekennzeichnet, dass**
der Verdampfungsbehälter (38, 138) ein Volumen aufweist, das wenigstens halb so groß, bevorzugt wenigstens gleich groß wie das Volumen eines flüssigen Druckgases in der Gaspatrone (42) ist, die zum Anschließen an das Anschlusselement (36, 136) vorgesehen ist oder die an das Anschlusselement (36, 136) angeschlossen ist.

4. Austragsvorrichtung nach einem der vorangehenden Ansprüche, **dadurch gekennzeichnet, dass**
die Verbindungselemente auf der gleichen Seite mit dem Verdampfungsbehälter (38, 138) verbunden sind.

5. Austragsvorrichtung nach einem der vorangehenden Ansprüche, **dadurch gekennzeichnet, dass**
der gasdurchlässige Kanal (35, 134, 135) gewunden ist, insbesondere zumindest zwei Richtungswechsel aufweist.

6. Austragsvorrichtung nach einem der vorangehenden Ansprüche, **dadurch gekennzeichnet, dass**
zwischen den Verbindungselementen eine gasundurchlässige Wandung im Grundkörper (1, 101) angeordnet ist.

7. Austragsvorrichtung nach einem der vorangehenden Ansprüche, **dadurch gekennzeichnet, dass**
bei einer normalen und so vorgesehenen Verwendung der Austragsvorrichtung zum Austragen einer fließfähigen Masse aus einer Kartusche (26) der Verdampfungsbehälter (38, 138) derart in der Austragsvorrichtung angeordnet ist, dass die beiden Öffnungen des gasdurchlässigen Kanals (35, 134, 135) in den Verdampfungsbehälter (38, 138) nicht bodenseitig mit dem Verdampfungsbehälter (38, 138) verbunden sind, so dass flüssige Bestandteile des Gasstroms sich bodenseitig im Verdampfungsbehälter (38, 138) sammeln, wobei bevorzugt die Öffnungen des gasdurchlässigen Kanals (35, 134, 135) in den Verdampfungsbehälter (38, 138) im oberen Bereich des Verdampfungsbehälters (38, 138) angeordnet sind, besonders bevorzugt in der Deckwandung des Verdampfungsbehälters (38, 138) angeordnet sind.

8. Austragsvorrichtung nach einem der vorangehenden Ansprüche, **dadurch gekennzeichnet, dass**
die Austragsvorrichtung ein Griffstück (8) aufweist, wobei der Verdampfungsbehälter (38, 138) zumindest bereichsweise in dem Griffstück (8) angeordnet ist, bevorzugt der Verdampfungsbehälter (38, 138) im Wesentlichen im Griffstück (8) angeordnet ist, besonders bevorzugt der Verdampfungsbehälter (38, 138) im Griffstück (8) angeordnet ist.

9. Austragsvorrichtung nach einem der vorangehenden Ansprüche, **dadurch gekennzeichnet, dass**
die Austragsvorrichtung eine Gaspatrone (42) beinhaltet, vorzugsweise eine Druckgaspatrone (42), insbesondere eine mit flüssigem Kohlendioxid gefüllte Gaspatrone (42).

10. Austragsvorrichtung nach einem der vorangehenden Ansprüche, **dadurch gekennzeichnet, dass**
am Anschlusselement (36, 136) ein Dichtungselement zum Anschließen der Gaspatrone (42) vorgesehen ist.

11. Austragsvorrichtung nach einem der vorangehenden Ansprüche, **dadurch gekennzeichnet, dass**
am Grundkörper (1, 101) eine Adaptereinheit (4, 6, 104) zum Anschließen von Kartuschen (26) angeordnet ist, wobei in dem Grundkörper (1, 101) ein zweiter und ein dritter gasdurchlässiger Kanal (30, 130) vorgesehen ist, die das Ventilelement (10, 110) mit der Außenseite der Adaptereinheit (4, 6, 104) verbinden.

12. Austragsvorrichtung nach Anspruch 11, **dadurch gekennzeichnet, dass**
zumindest ein Überdruckventil (28, 29) und/oder zumindest ein Sterilfilter mit dem dritten Kanal (30) verbunden ist oder über das Ventilelement (10, 110) verbindbar ist und das Überdruckventil (28, 29) und/oder der Sterilfilter über eine Öffnung im Grundkörper (1, 101) mit der umgebenden Atmosphäre verbunden ist, wobei die Öffnung nicht in der Adaptereinheit (4, 6, 104) angeordnet ist.

13. Austragsvorrichtung nach einem der vorangehenden Ansprüche, **dadurch gekennzeichnet, dass**
das Ventilelement (10, 110) einen ersten Gaskanal (12, 112) zum Regulieren des Druckgasstroms aufweist und bevorzugt zusätzlich einen zweiten Gaskanal aufweist, der so angeordnet ist, dass bei Betätigung des Ventilelements (10, 110) eine Unterbrechung des ersten Gaskanals (12, 112) erfolgt und der zweite Gaskanal geöffnet ist, so dass der zweite Gaskanal über den Grundkörper (1, 101) gasdurchlässig mit der umgebenden Atmosphäre verbunden ist.

14. Austragsvorrichtung nach einem der vorangehenden Ansprüche, **dadurch gekennzeichnet, dass**
der der Verdampfungsbehälter (38, 138) und/oder der Grundkörper (1, 101) aus Kunststoff besteht, bevorzugt aus Polyethylen.

15. Verwendung einer Austragsvorrichtung nach einem der vorangehenden Ansprüche zum Auspressen einer fließfähigen Masse aus einer Kartusche (26), bei dem ein verdampfendes flüssiges Gas aus einer Gaspatrone (42) in den Verdampfungsbehälter (38, 138) geleitet wird und das verdampfte Gas vom Verdampfungsbehälter (38, 138) über ein regulierbares Ventilelement (10, 110) zu einem Förderkolben (27) der Kartusche (26) geleitet wird, wobei der Gasdruck den Förderkolben (27) vortreibt und die fließfähige Masse durch den Vortrieb des Förderkolbens (27) aus der Kartusche (26) ausgetrieben wird.

## Claims

1. Dispensing device for the dispensation of flowable masses from cartridges (26), whereby the dispensing device comprises
a main body (1, 101),
a connecting element (36, 136) for a gas cartridge (42) on the main body (1, 101),
a valve element (10, 110) for regulation of a gas flow from the gas cartridge (42), and
a gas-permeable channel (35, 134, 135) that connects the connecting element (36, 136, 145) to the valve element (10, 110), **characterised in that**
at least one evaporation container (38, 138) is arranged in the gas-permeable channel (35, 134, 135) such that the gas flow from the gas cartridge (42) is guided through the evaporation container (38, 138), at least over regions thereof, whereby the gas-permeable channel (35, 134, 135) comprises two connecting elements that are connected to the evaporation container (38, 138) such that the gas flow from the gas cartridge (42) flows between the connecting elements through the evaporation container (38, 130), and
whereby the connecting elements are not connected to the evaporation container (38, 138) on opposite walls of the evaporation container (38, 138).

2. Dispensing device according to claim 1, **characterised in that**
the evaporation container (38, 138) has a larger cross-section than the gas-permeable channel (35, 134, 135), preferably a cross-section that is at least twice as large, particularly preferably a cross-section that it is at least ten times as large.

3. Dispensing device according to claim 1 or 2, **characterised in that**
the evaporation container (38, 138) comprises a volume that is at least half as large, preferably at least as large as the volume of a liquid compressed gas in the gas cartridge (42), which is provided for connection to the connecting element (36, 136) or which is connected to the connecting element (36, 136).

4. Dispensing device according to any one of the preceding claims, **characterised in that**
the connecting elements are connected to the evaporation container (38, 138) on the same side.

5. Dispensing device according to any one of the preceding claims, **characterised in that**
the gas-permeable channel (35, 134, 135) is tortuous, in particular comprises at least two changes of direction.

6. Dispensing device according to any one of the preceding claims, **characterised in that**
a gas-imprimatur wall is arranged between the connecting elements in the main body (1, 101).

7. Dispensing device according to any one of the preceding claims, **characterised in that**,
for a normal and intended use of the dispensing device for dispensation of a flowable mass from a cartridge (26), the evaporation container (38, 138) is arranged appropriately in the dispensation device such that the two openings of the gas-permeable channel (35, 134, 135) leading into the evaporation container (38, 138) are not connected on the floor side to the evaporation container (38, 138) such that liquid components of the gas flow collect on the floor side in the evaporation container (38, 138), whereby the openings of the gas-permeable channel (35, 134, 135) leading into the evaporation container (38, 138) preferably are arranged in the upper region of the evaporation container (38, 138), particularly preferably are arranged in the top wall of the evaporation container (38, 138).

8. Dispensing device according to any one of the preceding claims, **characterised in that**
the dispensation device comprises a handle (8), whereby at least regions of the evaporation container (38, 138) are arranged in the handle (8), preferably the evaporation container (38, 138) is essentially arranged in the handle (8), particularly preferably the evaporation container (38, 138) is arranged in the handle (8).

9. Dispensing device according to any one of the preceding claims, **characterised in that**
the dispensing device includes a gas cartridge (42), preferably a compressed gas cartridge (42), in particular a liquid carbon dioxide-filled gas cartridge (42).

10. Dispensing device according to any one of the preceding claims, **characterised in that**
a sealing element for connection of the gas cartridge (42) is provided on the connecting element (36, 136).

11. Dispensing device according to any one of the preceding claims, **characterised in that**
an adapter unit (4, 6, 104) for connection of cartridges (26) is arranged on the main body (1, 101), whereby a second and a third gas-permeable channel (30, 130) connecting the valve element (10, 110) to the outside of the adapter unit (4, 6, 104) are provided in the main body (1, 101).

12. Dispensing device according to claim 11, **characterised in that**
at least one overpressure valve (28, 29) and/or at least one sterile filter is connected to the third channel (30) or can be connected via the valve element (10, 110), and the overpressure valve (28, 29) and/or the sterile filter is/are connected to the ambient atmosphere via an opening in the main body (1, 101), whereby the opening is not arranged in the adapter unit (4, 6, 104).

13. Dispensing device according to any one of the preceding claims, **characterised in that**
the valve element (10, 110) comprises a first gas channel (12, 112) for regulation of the compressed gas flow and preferably comprises, in addition, a second gas channel that is arranged appropriately such that, upon actuation of the valve element (10, 110), the first gas channel (12, 112) is being interrupted and the second gas channel is being opened such that the second gas channel is connected in gas-permeable manner to the ambient atmosphere via the main body (1, 101).

14. Dispensing device according to any one of the preceding claims, **characterised in that**
the evaporation container (38, 138) and/or the main body (1, 101) consists of plastics, preferably of polyethylene.

15. Use of a dispensing device according to any one of the preceding claims for extruding a flowable mass from a cartridge (26), in which an liquid gas that can be evaporated is guided from a gas cartridge (42) into the evaporation container (38, 138) and the evaporated gas is guided from the evaporation container (38, 138) via a valve element (10, 110) that can be regulated to a feed plunger (27) of the cartridge (26), whereby the gas pressure propels the feed plunger (27) and the flowable mass is expelled from the cartridge (26) by the propulsion of the feed plunger (27).

## Revendications

1. Dispositif d'extraction pour l'extraction de masses coulantes de cartouches (26), dans lequel le dispositif d'extraction présente
un corps de base (1, 101),
un élément de raccord (36, 136) pour une cartouche de gaz (42) sur le corps de base (1, 101),
un élément de vanne (10, 110) pour la régulation d'un courant gazeux provenant de la cartouche de gaz (42) et
un canal perméable au gaz (35, 134, 135) qui connecte l'élément de raccord (36, 136, 145) à l'élément de vanne (10, 110), **caractérisé en ce que** au moins un récipient d'évaporation (38, 138) est disposé dans le canal perméable au gaz (35, 134, 135) de sorte que le courant gazeux provenant de la cartouche de gaz (42) est dirigé au moins par région à travers le récipient d'évaporation (38, 138), dans lequel le canal perméable au gaz (35, 134, 135) comprend deux éléments de connexion qui sont connectés au récipient d'évaporation (38, 138) de sorte que le courant gazeux provenant de la cartouche de gaz (42) s'écoule entre les éléments de connexion à travers le récipient d'évaporation (38, 138), et dans lequel les éléments de connexion ne sont pas connectés au récipient d'évaporation (38, 138) au niveau de parois opposées du récipient d'évaporation (38, 138).

2. Dispositif d'extraction selon la revendication 1, **caractérisé en ce que**
le récipient d'évaporation (38, 138) présente une section transversale supérieure au canal perméable au gaz (35, 134, 135), de préférence une section transversale au moins double, de manière particulièrement préférée une section transversale au moins dix fois aussi grande.

3. Dispositif d'extraction selon la revendication 1 ou 2, **caractérisé en ce que**
le récipient d'évaporation (38, 138) présente un volume qui est au moins deux fois moins grand, de préférence au moins aussi grand que le volume d'un gaz comprimé liquide dans la cartouche de gaz (42) qui est prévue pour le raccordement à l'élément de raccord (36, 136) ou qui est raccordée à l'élément de raccord (36, 136).

4. Dispositif d'extraction selon une des revendications précédentes, **caractérisé en ce que**
les éléments de connexion sont connectés au récipient d'évaporation (38, 138) sur le même côté.

5. Dispositif d'extraction selon une des revendications précédentes, **caractérisé en ce que**
le canal perméable au gaz (35, 134, 135) est sinueux, présente notamment au moins deux changements de sens.

6. Dispositif d'extraction selon une des revendications précédentes, **caractérisé en ce que**
une paroi imperméable au gaz est disposée dans le corps de base (1, 101) entre les éléments de connexion.

7. Dispositif d'extraction selon une des revendications précédentes, **caractérisé en ce que**
le récipient d'évaporation (38, 138) est disposé dans le dispositif d'extraction en cas d'utilisation normale et ainsi prévue du dispositif d'extraction pour l'extraction d'une masse coulante d'une cartouche (26) de telle sorte que les deux ouvertures du canal perméable au gaz (35, 134, 135) dans le récipient d'évaporation (38, 138) ne sont pas connectées du côté du fond au récipient d'évaporation (38, 138) de sorte que des constituants liquides du courant gazeux s'accumulent du côté du fond dans le récipient d'évaporation (38, 138), dans lequel les ouvertures du canal perméable au gaz (35, 134, 135) dans le récipient d'évaporation (38, 138) sont disposées de préférence dans la région supérieure du récipient d'évaporation (38, 138), sont disposées de manière particulièrement préférée dans la paroi supérieure du récipient d'évaporation (38, 138).

8. Dispositif d'extraction selon une des revendications précédentes, **caractérisé en ce que**
le dispositif d'extraction présente une poignée (8), dans lequel le récipient d'évaporation (38, 138) est disposé au moins par région dans la poignée (8), le récipient d'évaporation (38, 138) est disposé de préférence essentiellement dans la poignée (8), le récipient d'évaporation (38, 138) est disposé de manière particulièrement préférée dans la poignée (8).

9. Dispositif d'extraction selon une des revendications précédentes, **caractérisé en ce que**
le dispositif d'extraction contient une cartouche de gaz (42), de préférence une cartouche de gaz comprimé (42), notamment une cartouche de gaz (42) remplie de dioxyde de carbone liquide.

10. Dispositif d'extraction selon une des revendications précédentes, **caractérisé en ce que**
un élément d'étanchéité est prévu sur l'élément de raccord (36, 136) pour le raccordement de la cartouche de gaz (42).

11. Dispositif d'extraction selon une des revendications précédentes, **caractérisé en ce que**
un module adaptateur (4, 6, 104) est disposé sur le corps de base (1, 101) pour le raccordement de cartouches (26), dans lequel un deuxième et un troisième canal perméable au gaz (30, 130) sont prévus dans le corps de base (1, 101), lesquels connectent l'élément de vanne (10, 110) au côté externe du module adaptateur (4, 6, 104).

12. Dispositif d'extraction selon la revendication 11, **caractérisé en ce que**
au moins une soupape de sûreté (28, 29) et/ou au moins un filtre stérile est connecté(e) au troisième canal (30) ou peut être connecté(e) par le biais de l'élément de vanne (10, 110) et la soupape de sûreté (28, 29) et/ou le filtre stérile est connecté(e) à l'atmosphère environnante par le biais d'une ouverture dans le corps de base (1, 101), dans lequel l'ouverture n'est pas disposée dans le module adaptateur (4, 6, 104).

13. Dispositif d'extraction selon une des revendications précédentes, **caractérisé en ce que**
l'élément de vanne (10, 110) présente un premier canal de gaz (12, 112) pour la régulation du courant de gaz comprimé et présente de préférence en outre un second canal de gaz qui est disposé de sorte qu'en cas d'actionnement de l'élément de vanne (10, 110), une interruption du premier canal de gaz (12, 112) a lieu et le second canal de gaz est ouvert de sorte que le second canal de gaz est connecté à l'atmosphère environnante de manière perméable au gaz par le biais du corps de base (1, 101).

14. Dispositif d'extraction selon une des revendications précédentes, **caractérisé en ce que**
le récipient d'évaporation (38, 138) et/ou le corps de base (1, 101) se compose de plastique, de préférence de polyéthylène.

15. Utilisation d'un dispositif d'extraction selon une des revendications précédentes pour le pressage d'une masse coulante d'une cartouche (26), dans lequel un gaz liquide s'évaporant provenant d'une cartouche de gaz (42) est conduit dans le récipient d'évaporation (38, 138) et le gaz évaporé est conduit du récipient d'évaporation (38, 138) par le biais d'un élément de vanne pouvant être régulé (10, 110) vers un piston d'alimentation (27) de la cartouche (26), dans laquelle la pression gazeuse pousse le piston d'alimentation (27) et la masse coulante est expulsée de la cartouche (26) par la poussée du piston d'alimentation (27).
